# FASCICULE DE BREVET EUROPEEN

(11) **EP 2 477 677 B1**
(45) Date de publication et mention de la délivrance du brevet: **17.01.2018**
(21) Numéro de dépôt: 10768567.9
(22) Date de dépôt: 13.09.2010
(51) Int. Cl.: A61M 5/00, A61M 5/14

(54) **DISPOSITIF POUR L'INJECTION DE PRODUITS DE CONTRASTE**
VORRICHTUNG ZUM INJIZIEREN VON KONTRASTMITTELN
DEVICE FOR INJECTING CONTRAST PRODUCTS

(30) Priorité: 18.09.2009 EP 09170730
(43) Date de publication de la demande: 25.07.2012
(73) Titulaire: Bracco Injeneering SA, 1004 Lausanne (CH)
(72) Inventeur: DUFFOUR, Hervé, 1004 Lausanne (CH); NEFTEL, Frédéric, 1005 Lausanne (CH)
(74) Mandataire: Poletti, Marco
(86) Numéro de dépôt international: PCT/IB2010/054114
(87) Numéro de publication internationale: WO 2011/033440

(56) Documents cités:
- EP-A1- 1 867 356
- US-A1- 2007 213 848

## Description

### Domaine de l'invention

Le domaine de l'invention est celui de l'imagerie médicale, obtenue par injection de produits de contrastes et, plus précisément, celui de l'injection de produits de contrastes plus ou moins dilués tels que pour l'imagerie par Rx et scanner CT ou celui de l'imagerie IRM.

### Etat de la technique

On connaît déjà aujourd'hui l'utilisation d'injecteurs pousse-seringues capables d'injecter depuis deux seringues en parallèles un produit de contraste simultanément à une solution saline, ceci afin de diluer le produit de contraste. Les brevets US 7267667, ainsi que US 5911252 décrivent de tels dispositifs.

US 2007/213848 il décrit un mécanisme d'injection alternée (valve off/on) d'un composé radioactif dans une solution saline. Ce mécanisme concerne donc une méthode d'élution (et non de dilution) du composé radioactif dans une solution saline. Cette méthode d'élution - plus précisément du contrôle de l'élution - permet de contrôler l'activité du composé radioactif. Si cette dernière est trop faible au niveau du détecteur, la solution saline pénètre dans le réservoir contenant le composé radioactif et en prélève une certaine quantité par élution. Dès que l'activité mesurée par le détecteur retrouve la valeur souhaitée, la valve se ferme et la solution saline ne traverse que la conduite « by-pass ».

### Exposé général de l'invention

Dans l'invention, la solution du problème précité consiste à injecter du produit de contraste dans un organe à analyser de telle façon que les concentrations du tel produit de contraste varient au court du temps et/ou de la région de l'organe analysé au moment de l'acquisition des images.

La présente invention présente plusieurs avantages, en particulier de pouvoir réaliser de telles modifications de concentration dans l'organe à un moment précis correspondant à l'acquisition des images, sans avoir recours à deux injecteurs utilisés en parallèles, à un moindre coût et avec un moindre risque (notamment d'erreur et/ou de contamination) pour le patient.

Il convient de relever que la présente invention diffère de l'enseignement de US 2007/213848 en ce que l'on fait varier la concentration du produit de contraste, en alternant les injections « solution saline/produit de contraste » de manière à ce que le mélange - donc la concentration souhaitée - soit obtenu avant l'arrivée dans le coeur.

L'invention peut en outre être appliquée à l'amplification d'un signal de détection d'extravasation, par exemple par ultra-sons, situé en aval du point d'injection du patient du fait du changement de la nature ou la concentration du liquide injecté. Ainsi il est possible de s'assurer que le liquide soit bien injecté à l'intérieur des vaisseaux concernés et d'exclure de la sorte toute extravasation éventuelle en cas de retrait de l'aiguille d'injection de la veine du patient pendant ladite injection. Dans le cas d'une utilisation d'un détecteur d'extravasation, le signal étant plus ou moins constant, on peut parfois ne plus être à même de s'assurer de la mesure correcte du signal (faux positif, voire faux négatif). Par la modification en cours d'injection de la nature du liquide (correspondant à un changement du signal mesuré) et l'éventuelle correspondance de ce changement mesuré par rapport à la séquence d'injection, on peut s'assurer du bon fonctionnement du détecteur d'extravasation et en déduire un flux normal d'injection qui exclut toute extravasation. A défaut de mesurer ce signal variable en fonction de la fréquence d'injection des deux liquides alternés, on peut en déduire soit un mauvais positionnement de la sonde de mesure, soit une extravasation et, dans les deux cas, interrompre l'injection.

### Exposé détaillé de l'invention

L'invention est décrite plus en détail ci-après au moyen d'exemples illustrés par les figures suivantes :

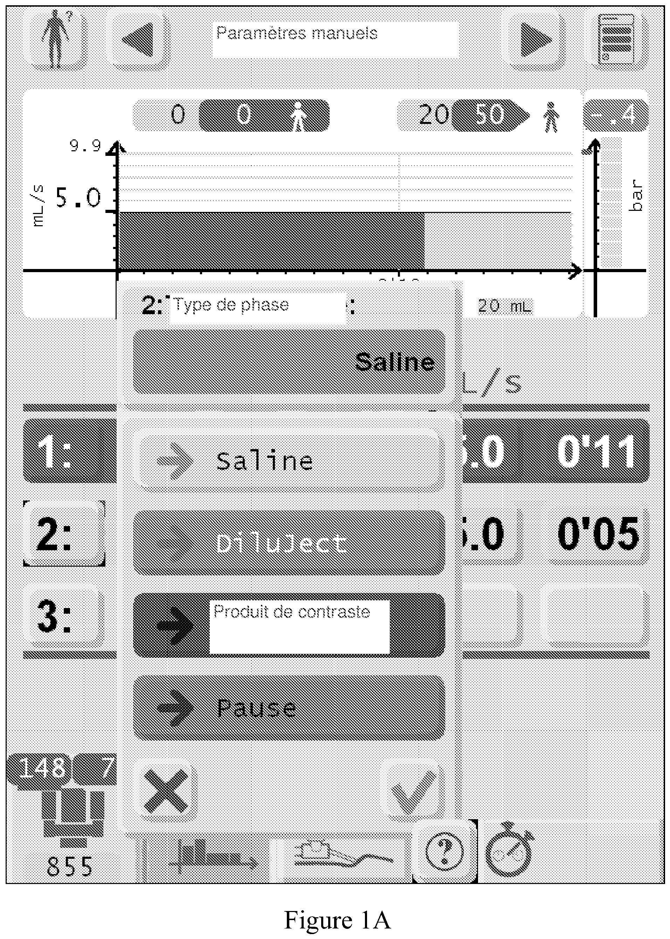

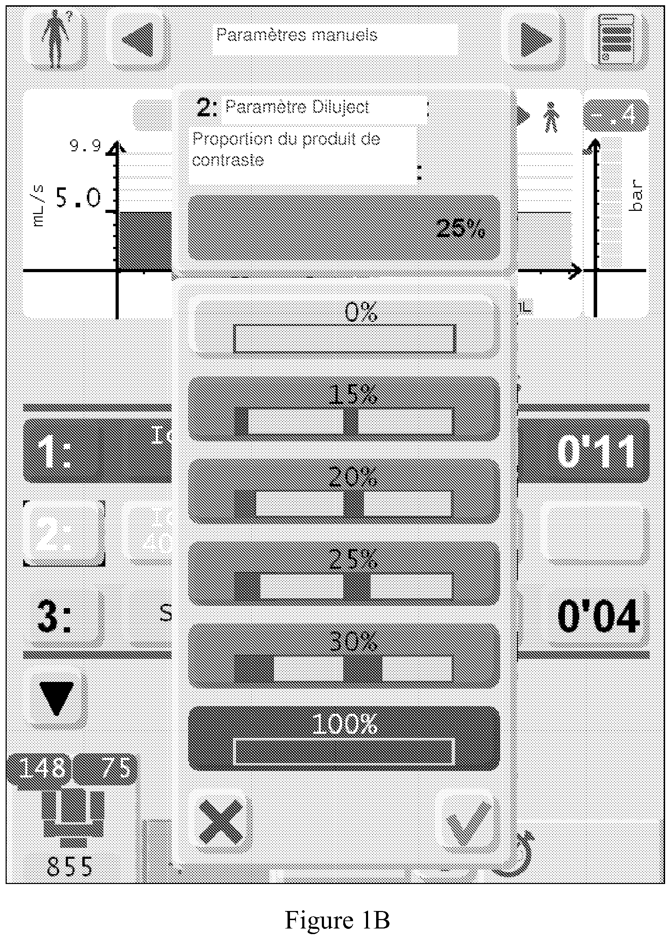

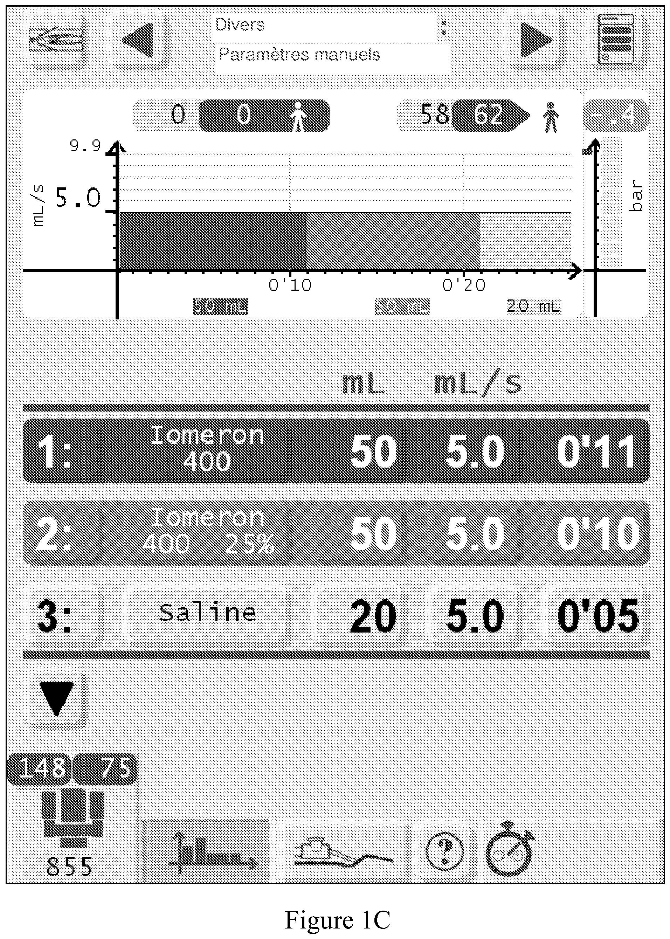

Les figures 1A, 1B et 1C présentent un exemple d'interface de programmation de l'injecteur, figurant les différentes concentrations possibles avec en Figure 1A l'option de choix entre l'injection de contraste, de saline ou de dilution « Diluject »; en Figure 1B les possibilités de dilution (15%, 20%, 25% ou 30%) ; et en figure 1C la phase de dilution sur un diagramme.

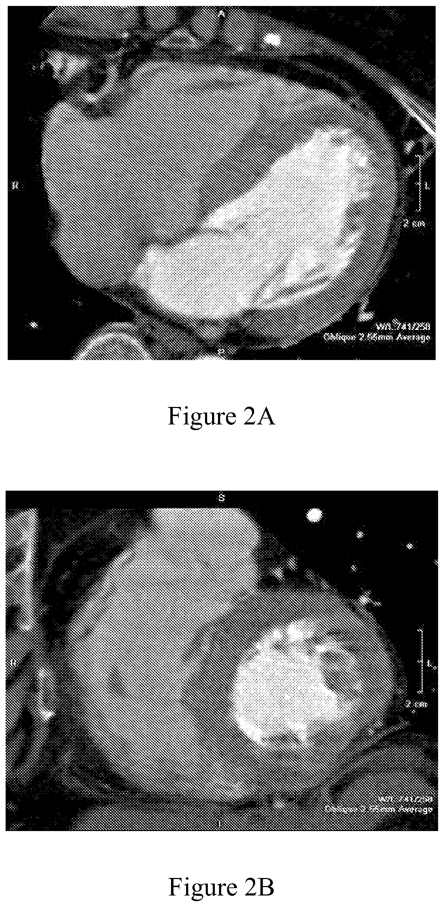

Les figures 2A et 2B représentent des images obtenues avec une dilution de 15% et un débit de 4ml/s (2A et 2B) et montrent une dilution optimale dans le coeur avec une différence, dans cet exemple, entre la concentration dans le coeur droit et le coeur gauche.

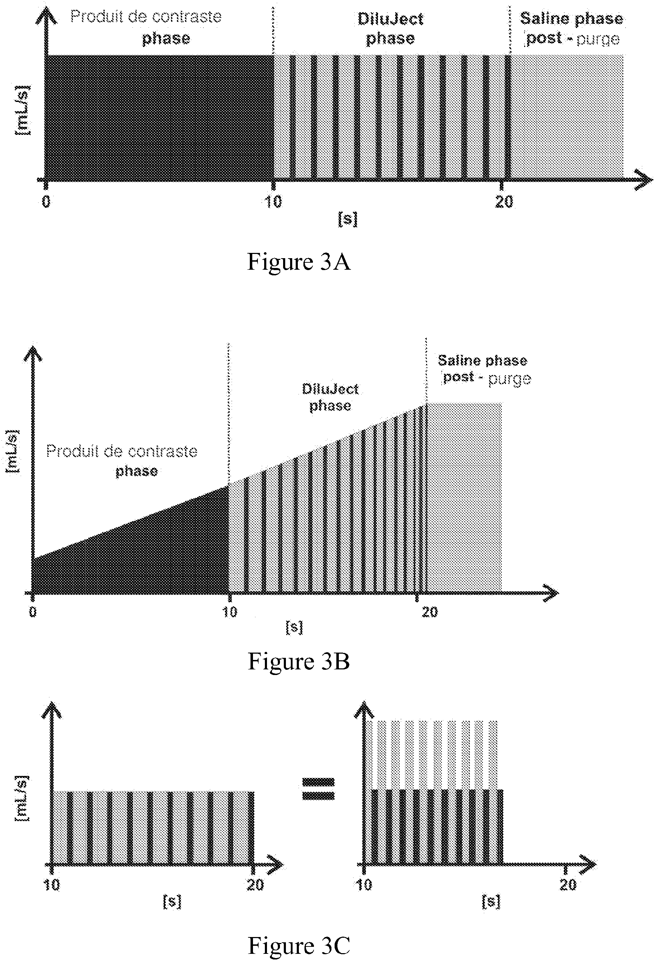

Les figures 3A-3C (3A dilution fixe, 3B dilution à débit et fréquence variable, 3C deux exemples de dilution à débits différents) représentent des exemples de cycles d'injection avec différents modes de dilution.

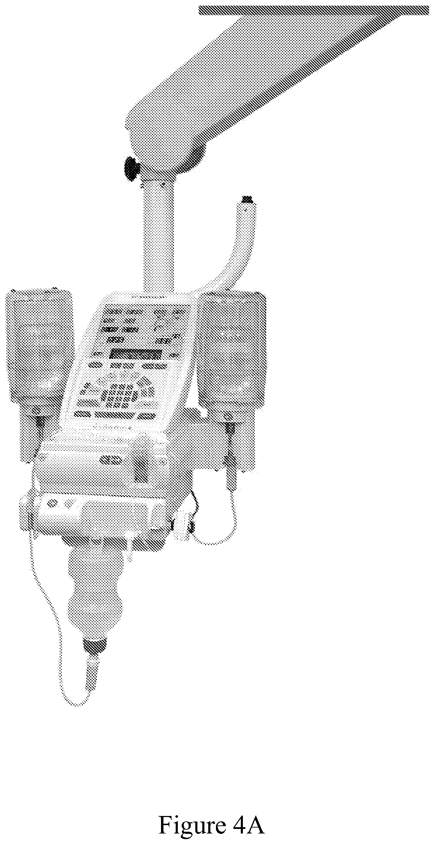

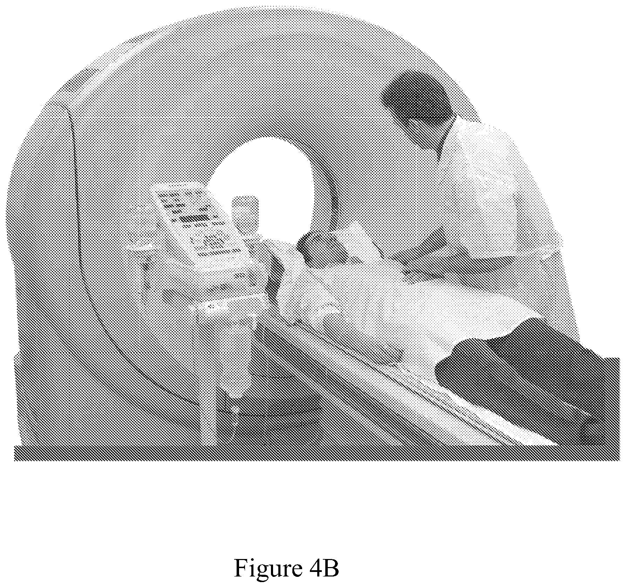

Les figures 4A et 4B représentent un injecteur permettant la réalisation de l'invention.

Un des objectifs de la présente invention est d'obtenir une dilution du produit de contraste (soit en utilisant deux produits de contrastes différents, soit en utilisant un produit de contraste et une solution saline ou toute autre solution qui n'a pas d'effet sur le contraste au cours de l'examen tel qu'une solution pharmaceutique non iodée) avant l'arrivée dans l'organe cible (par exemple le coeur droit du patient), afin de pouvoir améliorer l'acquisition d'informations relatives à l'anatomie et au fonctionnement de l'organe à analyser (dans le cas du coeur : visualisation du septum, des coronaires, meilleur calcul de la fraction d'éjection du coeur ; sachant que pour se faire le coeur gauche est préférentiellement rempli avec du contraste pendant que le coeur droit est rempli avec une solution de contraste diluée), soit au même moment soit au moment respectif ou les acquisitions des images se font respectivement dans l'organe en question (d'abord le coeur gauche, puis le coeur droit).

Pour ce faire l'invention consiste de préférence à injecter successivement dans la veine du patient des phases de produit de contraste alternées avec des phases d'injection de solution saline ou d'un produit de contraste plus faiblement concentré, ceci afin d'obtenir un mélange entre les différentes phases qui puisse se faire dans le système cardiovasculaire du patient avant l'arrivée dans le coeur ou l'organe destiné à être analysé. L'invention peut être avantageusement utilisée dans l'imagerie par Scanner CT ou dans l'IRM. Pour obtenir une dilution efficace il faut que la fréquence soit suffisamment rapide afin que le mélange puisse se faire dans le système cardiovasculaire du patient avant l'arrivée dans l'organe visé. Pour le coeur, il est souhaitable d'avoir une fréquence assez proche de celle de la fréquence cardiaque, typiquement de l'ordre de 1 Hz, mais cela peut également être efficace entre 5Hz et 0.2 Hz.

Ainsi on peut se passer d'un double injecteur et réaliser la dilution directement dans le corps du patient avec un seul injecteur relié à deux types de solutions de concentrations différentes (ou une solution de contraste et une solution saline) à un coût moindre et nécessitant moins de connections et de manipulations qui représentent toujours un risque en terme d'asepsie et d'erreurs.

Typiquement on peut choisir le pourcentage souhaité de dilution à obtenir dans le coeur (p.ex. 15%, 20%, 25% ou 30% de dilution, c'est à dire pour 25% qu'il y aura 25% de produit de contraste seulement et 75% de solution saline). Dans le cas d'une dilution à 25%, on peut choisir par exemple une phase de 1ml de contraste suivie d'une phases de 3ml de saline, les deux phases représentant un cycle qui est répété en fonction du volume total que l'on souhaite injecter (p. ex. pour 20ml injecté on réalisera 5 cycles successifs dans l'exemple décrit). Le débit est, dans ce cas, le même pour la solution saline et la solution de contraste (p. ex. 4ml/s). Alternativement on peut aussi faire varier le débit afin d'obtenir le même effet de dilution avec un volume modifié. Ainsi dans l'exemple décrit plus haut, on peut remplacer les 3ml de la phase de solution saline par une phase de 1.5ml avec un débit réduit de moitié (dans l'exemple 2ml/s au lieu des 4ml/s). On peut aussi choisir de faire varier le débit de la solution de contraste et réaliser d'autres combinaisons ayant pour effet de modifier les rapports de contraste dans l'organe ciblé. On peut également choisir de faire varier les débits et/ou volumes respectifs de saline et de contraste à chaque cycle, par exemple de façon progressive, afin d'obtenir des images dynamiques avec des concentrations variables au cours du temps d'examen et/ou d'acquisition de l'image, selon des algorithmes pouvant revêtir toute forme mathématique utile.

Afin de réaliser un tel dispositif, on utilise de préférence un processeur capable de gérer l'alternance entre les deux réservoirs, par exemple en commandant l'ouverture et la fermeture de clamps situés entre chacun des réservoirs et le dispositif d'injection (par exemple une cassette péristaltique).

## Revendications

1. Dispositif médical pour l'injection de produits de contraste comprenant au moins deux réservoirs séparés dont les contenus respectifs ne peuvent se mélanger à l'intérieur de l'un et/ou l'autre desdits réservoirs, un injecteur et un distributeur disposé de manière à mettre en communication de manière alternée lesdits réservoirs avec ledit injecteur, chaque réservoir etánt separement en communication fluidique avec ledit injecteur, dispositif médical **caractérisé par le fait qu'**il comprend des moyens pour assurer ladite communication alternée à une fréquence comprise entre 0.2 et 5 Hz pendant au minimum deux cycles consécutifs, et que un mélange dilué desdits contenus est obtenu avant l'arrivée dans le coeur ou l'organe destiné à être analysé.

2. Dispositif médical selon la revendication 1 dans lequel la fréquence est d'environ 1 Hz.

3. Dispositif médical selon la revendication 1 dans lequel la fréquence est variable au cours du temps.

4. Dispositif médical selon la revendication 1 à 3 adapté pour autoriser un écoulement continu compris entre 0.1 à 5 ml avec le même réservoir.

5. Dispositif médical selon l'une des revendications précédentes dans lequel un réservoir contient un produit de contraste et l'autre réservoir contient une solution saline.

6. Dispositif médical selon l'une des revendications précédentes 1 à 4 dans lequel chaque réservoir comporte un produit de contraste avec une concentration qui lui est spécifique.

## Patentansprüche

1. Medizingerät zum Injizieren von Kontrastmitteln, das mindestens zwei getrennte Behälter, deren jeweiliger Inhalt sich nicht im Inneren des einen und/oder des anderen der Behälter vermischen kann, einen Injektor und ein Ventil umfasst, das so angeordnet ist, dass es abwechselnd die Behälter mit dem Injektor in Verbindung bringt, wobei jeder Behälter separat mit dem Injektor fluidverbunden ist, wobei das Medizingerät **dadurch gekennzeichnet ist, dass** es Mittel zum Gewährleisten der abwechselnden Verbindung mit einer Frequenz zwischen 0,2 und 5 Hz während mindestens zwei aufeinanderfolgender Zyklen umfasst, und dass eine verdünnte Mischung des Inhalts vor dem Erreichen des Herzens oder des zu untersuchenden Organs erhalten wird.

2. Medizingerät nach Anspruch 1, wobei die Frequenz ungefähr 1 Hz beträgt.

3. Medizingerät nach Anspruch 1, wobei die Frequenz zeitlich veränderlich ist.

4. Medizingerät nach Anspruch 1 bis 3, das so ausgelegt ist, dass es einen kontinuierlichen Fluss zwischen 0,1 und 5 ml mit demselben Behälter zulässt.

5. Medizingerät nach einem der vorhergehenden Ansprüche, wobei ein Behälter ein Kontrastmittel enthält und der andere Behälter eine Kochsalzlösung enthält.

6. Medizingerät nach einem der vorhergehenden Ansprüche 1 bis 4, wobei jeder Behälter ein Kontrastmittel mit einer spezifischen Konzentration aufweist.

## Claims

1. Medical device for injecting contrast media and comprising at least two separate reservoirs the respective contents of which cannot mix with one another in either one of said reservoirs, an injector and a directional control valve arranged in such a way as to place said reservoirs alternately in communication with said injector, each reservoir being separately in fluidic communication with said injector, said medical device being **characterized in that** it comprises means for bringing about said alternating communication at a frequency of between 0.2 and 5 Hz for a minimum of two consecutive cycles, and **in that** a diluted mixture of said contents is obtained before it reaches the heart or organ that is to be analyzed.

2. Medical device according to Claim 1, in which the frequency is about 1 Hz.

3. Medical device according to Claim 1, in which the frequency can vary over time.

4. Medical device according to Claims 1 to 3 and designed to allow continuous flow of between 0.1 and 5 ml with the same reservoir.

5. Medical device according to one of the preceding claims, in which one reservoir contains a contrast medium and the other reservoir contains a saline solution.

6. Medical device according to one of the preceding Claims 1 to 4, in which each reservoir contains a contrast medium at a concentration specific to it.
